# EUROPÄISCHE PATENTSCHRIFT

(11) **EP 0 628 297 B1**
(45) Veröffentlichungstag und Bekanntmachung des Hinweises auf die Patenterteilung: **13.08.1997**
(21) Anmeldenummer: 94108418.8
(22) Anmeldetag: 01.06.1994
(51) Int. Cl.: A61F 5/01

(54) **Kniegelenksbandage**
Knee joint bandage
Genouillère

(30) Priorität: 08.06.1993 DE 4319011
(43) Veröffentlichungstag der Anmeldung: 14.12.1994
(73) Patentinhaber: SCHÜTT & GRUNDEI ORTHOPÄDIETECHNIK GmbH, D-23556 Lübeck (DE)
(72) Erfinder: Grundei, Hans, Dr., D-23558 Lübeck (DE); Etspüler, Rolf, Dr., D-23562 Lübeck (DE); Schmelzer, Ulrich, Dr., D-23568 Lübeck (DE)
(74) Vertreter: Fuchs, Luderschmidt & Partner Patentanwälte

(56) Entgegenhaltungen:
- DE-A- 3 637 879
- US-A- 4 116 236
- US-A- 4 201 203
- US-A- 4 986 263
- US-A- 5 016 621

## Beschreibung

Die Erfindung betrifft eine Kniegelenksbandage. Die Bandagen kommen zum Einsatz bei Patienten, die z. B. X-Beine haben, bei denen die Patella zu Luxationen nach lateral neigt.

Bekannte Kniegelenksbandagen bestehen aus einem röhrenförmigen Gebilde mit einer Kniekappe, welche eine Aussparung für die Patella aufweist.

Derartige bekannte Bandagen erfüllen ihren Zweck nur in engen Grenzen. Insbesondere vermögen sie nicht, eine Luxation der Patella nach lateral zu verhindern.

Bei einer aus der US-A-4,445,505 bekannt gewordenen Kniegelenksbandage sind zwei Kissen vorgesehen, die in Taschen angeordnet sind, um eine Luxation der Patella nach lateral zu vermeiden. Die Kissen sollen aus einem relativ steifen, aber noch nachgiebigem Plastikmaterial wie Polyethylenschaum bestehen. Hierbei wird als nachteilig angesehen, daß die Kissen nur in bezug auf die Patella fixiert sind durch Anordnung der sie aufnehmenden Taschen, nicht hingegen in bezug auf den Unterschenkel. Dies soll erst erzielt werden durch den Einsatz von Stützstreben, die jeweils auf beiden Seiten der Patella in den strumpfförmigen Grundkörper eingelassen sind.

Eine weitere Kniegelenksbandage ist bekannt geworden aus der US-A-4,116,236. Die darin beschriebene Bandage sieht vor, daß die Kniescheibe von einem kreisförmigen Kissen oder von einem Kissen in der Gestalt eines umgekehrten "U" umgeben ist, die der Kniescheibe Halt gibt. Auch hierbei ist die Positionierung in bezug auf Femur und Tibia nicht Gegenstand der Ausgestaltung des Kissens. Dies wird erst - wie schon in der vorerwähnten Druckschrift - erzielt durch dünne, nicht elastische Streifen, die vom oberen Abschnitt zum unteren Abschnitt des strumpfförmigen Grundkörpers verlaufen. Wie schon im Falle der erstgenannten Druckschrift findet also eine Entkoppelung statt zwischen der exakten Positionierung der Bandage als Ganzem und der Verhinderung der seitlichen Luxation der Patella.

Aus der US-A 5 016 621 ist eine Kniegelenksbandage bekannt geworden, die aus elastischem Material besteht und eine im wesentlichen röhrenförmige Gestalt aufweist. Die Patella wird in ihre Lage festgelegt durch mehrere Bänder, die zwischen dem oberen und dem unteren Teil der Bandage gespannt sind. In wenigstens eine mehr oder weniger vordere Tasche ist ein Element einschiebbar, welches die Lage der Patella dort stabilisieren soll. Aber auch bei dieser Kniegelenksbandage tritt das bereits geschilderte Phänomen der Entkoppelung zwischen der Positionierung der Bandage und der Luxationsverhinderung der Patella auf.

Vor diesem Hintergrund ist es die Aufgabe der vorliegenden Erfindung, eine Kniegelenksbandage anzugeben, welche die angesprochene Luxation der Patella nach lateral sicher vermeiden hilft, wobei eine funktionelle Einheit für die eigentliche Luxationsprävention und die richtige Positionierung der Bandage sorgen soll.

Gelöst wird die Aufgabe durch Kniegelenksbandage mit den Merkmalen des Anspruchs 1. Weitere vorteilhafte Ausgestaltungen ergeben sich aus den Unteransprüchen.

Demgemäß besteht die Kniegelenksbandage aus elastischem Material mit im wesentlichen röhrenförmiger Gestalt.

Ferner ist sie mit einer Tasche im vorderen Bereich zur Aufnahme eines die Lage der Patella stabilisierenden Elements versehen. Sie weist im Bereich der Patella einen Ausschnitt auf. In die vordere Tasche ist erfindungsgemäß ein steifes Kunststoffelement als stabilisierendes Element für die Patella einschiebbar. Dieses weist in seiner Breite eine Gestalt auf, die dem Unterschenkel nachempfunden und im oberen Bereich dem Ausschnitt für die Patella angepaßt ist.

Im lateralen, d. h. äußeren Bereich ist das Kunststoffelement gegenüber seinem medialen Bereich erhöht. Die Patella wird also teilweise vom Kunststoffelement im angelegten Zustand der Bandage umfaßt, und zwar so, daß die Luxation der Patella nach lateral sicher verhindert wird.

Vorteilhaft ist die Ausführung der Bandage, bei der das Kunststoffelement im Bereich der Patella von medial nach lateral im wesentlichen einem Kreisbogen folgt. Dies entspricht in etwa der anatomischen Gestalt der Patella.

Vorzugsweise ist darüber hinaus ein elastischer Zuggurt vorgesehen, der um die Bandage herum spannbar ist. Der Zuggurt erhöht die Stabilität der Bandage und damit deren Antiluxationswirkung.

Der Zuggurt ist vorzugsweise mittels eines Schnellverschlusses an der Bandage befestigbar und mit einem weiteren Schnellverschluß verschließbar. Zur Befestigung an der Bandage ist besonders bevorzugt daran ein Teil eines Klettenverschlusses angenäht, dessen zweiter Teil an dem einen Ende des Zuggurtes sitzt. Der zweite Schnellverschluß ist ebenfalls vorzugsweise ein Klettenverschluß, dessen eines Teil auf der anderen Seite des einen Endes sitzt, während das andere Teil auf dem anderen Ende des Gurtes befestigt ist.

Zum Anliegen streift der Patient die erfindungsgemäße Bandage über seinen Unterschenkel und weiter über sein Knie, bis das Kunststoffelement die Patella im beschriebenen Sinne teilweise umfaßt oder einfaßt. Sodann kann er gegebenenfalls den vorhandenen elastischen Zuggurt spannen und verschließen. Dabei sollte der Gurt kurz oberhalb des Knies am unteren Oberschenkel angebracht werden.

Die Erfindung wird anhand eines Ausführungsbeispiels gemäß der Zeichnungsfiguren naher erläutert. Hierbei zeigt:
- Fig. 1: die Vorderansicht auf ein Bein, mit angelegter erfindungsgemäßer Bandage, und
- Fig. 2: die Ansicht auf das Bein von lateral.

Die Kniebandage aus elastischem Material weist einen Ausschnitt 2 für die Patella 3 auf. Nach dem Anlegen liegt die Patella 3 also frei im Ausschnitt 2.

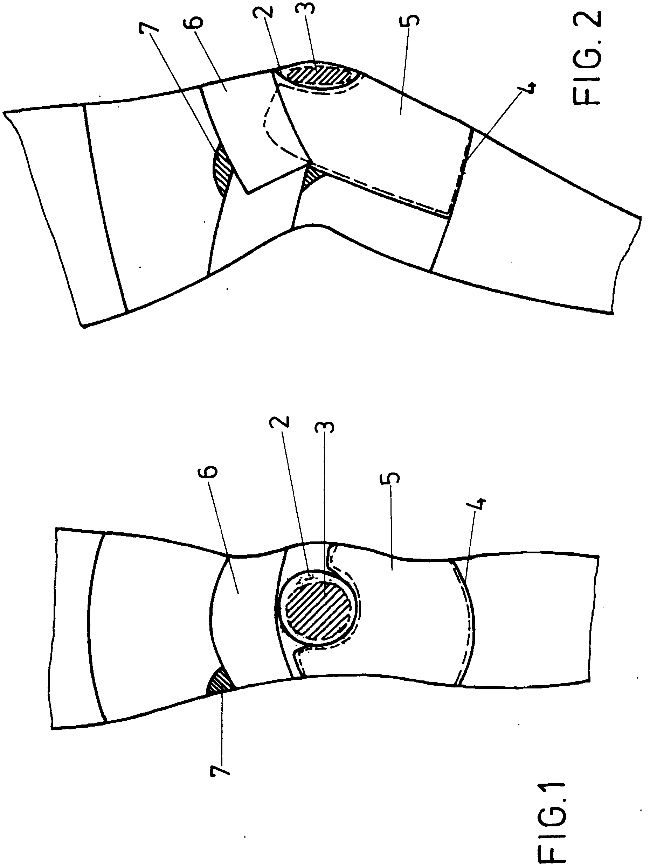

Es ist eine Tasche 4 vorgesehen, in welche das oben beschriebene steife Kunststoffelement 5 eingeschoben ist. Wie deutlich aus Fig. 1 ersichtlich, ist das Kunststoffelement gekrümmt ausgebildet, so daß es sich dem oberen Unterschenkel gut anpassen kann. Es ist im oberen Bereich so ausgebildet, daß es die Patella 3 teilweise einfaßt, derart, daß die Patella an einer Luxation nach lateral, in Fig. 1 also nach links, gehindert wird. Hierzu ist das Kunststoffelement 5 so ausgebildet, daß es lateral gegenüber der medialen Seite überhöht ist.

Aus Fig. 2 ist im übrigen die schalenförmige Ausbildung des Kunststoffelementes 5 ersichtlich.

Im dargestellten Ausführungsbeispiel ist im übrigen ein elastischer Zuggurt 6 vorgesehen, der mittels eines Schnellverschlusses, von dem das eine Teil 7 an der Bandage befestigt dargestellt ist, an dieser befestigbar ist. Der Zuggurt ist spannbar und an seinen Enden verschließbar. Er verleiht der Bandage eine höhere Stabilität.

## Patentansprüche

1. Kniegelenksbandage aus elastischem Material mit im wesentlichen röhrenförmiger Gestalt, mit einem Ausschnitt im Bereich der Patella und mit einer Tasche im vorderen Bereich zur Aufnahme eines die Lage der Patella stabilisierenden Elementes,
dadurch gekennzeichnet,
daß das stabilisierende Element ein in die vordere Tasche (4) einschiebbares, schalenförmiges und steifes Kunststoffelement (5) ist, das in seiner Breite eine dem Unterschenkel nach empfundene Gestalt aufweist, im oberen Bereich dem Ausschnitt (2) für die Patella (3) angepaßt ist und das im lateralen Bereich höher gegenüber seinem medialen Abschnitt ausgebildet ist.

2. Kniegelenksbandage nach Anspruch 1, bei der das Kunststoffelement (5) im Bereich der Patella (3) so ausgebildet ist, daß es medial nach lateral im wesentlichen einem Kreisbogenausschnitt folgt.

3. Kniegelenksbandage nach Anspruch 1 oder 2, bei der ein elastischer Zuggurt (6) vorgesehen ist, der um die Bandage herum spannbar ist.

4. Kniegelenksbandage nach Anspruch 3, bei der der Zuggurt (6) mittels eines Schnellverschlusses (7) an der Bandage befestigbar und mit einem weiteren Schnellverschluß verschließbar ist.

## Claims

1. Knee joint bandage of elastic material with essentially tubular shape, having an opening in the region of the patella and having a pocket in the front region to accommodate an element which stabilises the position of the patella, characterised in that the stabilising element is a bowl-shaped and stiff plastic element (5) which can be slid into the front pocket (4), and its width has a shape corresponding to the lower leg, its upper region is matched to the opening (2) for the patella (3) and the lateral region is designed to be higher with respect to its medial section.

2. Knee joint bandage according to claim 1, in which the plastic element (5) in the region of the patella (3) is designed so that it essentially follows an arc opening medially to laterally.

3. Knee joint bandage according to claim 1 or 2, in which an elastic tension belt (6) is provided which can be tightened around the bandage.

4. Knee joint bandage according to claim 3, in which the tension belt (6) can be attached to the bandage by means of a rapid closure (7) and can be closed using a further rapid closure.

## Revendications

1. Genouillère formée d'un matériau élastique possédant une configuration de forme essentiellement tubulaire, comportant une découpe au niveau de la rotule et une poche située dans la partie avant et servant à loger un élément stabilisant la position de la rotule,
caractérisé en ce
que l'élément stabilisateur est un élément en matière plastique rigide en forme de coque (5), qui peut être inséré dans la poche avant (4) et qui possède, sur son étendue en largeur, une configuration s'adaptant à la partie inférieure de la jambe et, dans sa partie supérieure, est adapté à la découpe (2) prévue pour la rotule (3) et, dans sa partie latérale, est disposé à un niveau plus élevé que dans sa partie médiane.

2. Genouillère selon la revendication 1, dans laquelle dans la zone de la rotule (3) l'élément en matière plastique (5) est agencé de telle sorte que depuis sa partie médiane en direction de sa partie latérale, il suit essentiellement une section d'arc de cercle.

3. Genouillère selon la revendication 1 ou 2, dans laquelle il est prévu une sangle élastique de traction (6) qui peut être tendue autour de la genouillère.

4. Genouillère selon la revendication 3, dans laquelle la sangle de traction (6) peut être fixée à la genouillère à l'aide d'un système de fermeture rapide (7) et peut être fermée par un autre système de fermeture rapide.
